# EUROPEAN PATENT SPECIFICATION

(11) **EP 3 346 957 B1**
(45) Date of publication and mention of the grant of the patent: **03.03.2021**
(21) Application number: 16775026.4
(22) Date of filing: 12.09.2016
(51) Int. Cl.: A61F 2/04

(54) **ENDOSCOPICALLY DEPLOYABLE ANTI-REFLUX DEVICE**
ENDOSKOPISCH EINSETZBARE ANTI-REFLUX-VORRICHTUNG
DISPOSITIF ANTI-REFLUX À DÉPLOIEMENT ENDOSCOPIQUE

(30) Priority: 11.09.2015 US 201562217329 P
(43) Date of publication of application: 18.07.2018
(73) Proprietor: Parker-Hannifin Corp, Cleveland, OH 44124 (US)
(72) Inventor: BANCO, Gino G., Lyndhurst, Ohio 44124 (US); COLLINSON, Michael, Camarillo, California 93102 (US); DEAN, Casey, Ventura, California 93004 (US); CARROLL, Derek, Los Angeles, California 90039 (US); ZARNEGAR, Rasa, New York, New York 10021 (US); CRAWFORD, Carl V., New York, New York 10029 (US); FINNERTY, Brendan, New York, New York 10021 (US); CIERCIEREGA, Thomas, Wyckoff, New Jersey 07481 (US)
(74) Representative: McDougall, James
(86) International application number: PCT/US2016/051245
(87) International publication number: WO 2017/044929

(56) References cited:
- EP-A1- 2 752 170
- WO-A1-2007/053556
- WO-A1-2011/073970
- WO-A1-2013/066617
- US-A1- 2002 032 487
- US-B1- 6 254 642

## Description

### Field of Invention

The present invention relates to stents and in particular to esophageal stents including a valve and an anchoring structure.

### Background

Gastroesophageal reflux disease (GERD), a condition in which stomach acid enters the esophagus, affects over 40,000,000 Americans yearly. Significant discomfort and/or damage can be caused to a patient experiencing reflux, as cells on the lining of the esophagus are not resistant to stomach acid. These patients are often treated with long-term antireflux medications. Some patients also do not respond to medications or become refractory to medical management, requiring further intervention and surgery. Subsets of patients undergo Nissen fundoplication procedures that recreate the valve at the distal esophagus to prevent reflux.

Currently, diagnosing reflux is challenging. Most patients are treated with medications after empirical examination. If they respond, the patients are left on medications indefinitely. Some patients undergo endoscopy, manometry, barium swallow radiograph, esophageal biopsy, and/or pH studies to evaluate their condition. Only endoscopy and pH measurement systems, however, can objectively establish the extent of reflux. Furthermore, only patients with chronic reflux for extended periods generally exhibit endoscopic evidence of reflux. As such, this visual technique is only effective as a diagnostic tool for a subset of patients, when damage has often already occurred. Measurement of pH in the esophagus also has drawbacks as a diagnostic tool.

Generally, there are two ways patients are evaluated during a pH study. The traditional method involves a 24-hour study during which a probe is placed in their esophagus, with trailing leads exiting the nostril and taped to the face. This can be uncomfortable and embarrassing for the patient, leading to a relatively short evaluation period in which the patient may not be participating in their normal activities. A more recent method involves a 48-hour study using a Medtronic Bravo system, in which a wireless probe is endoscopically placed in the distal esophagus. The probe sends information to a recorder via Bluetooth® technology. The Bravo system has an advantage in that it is the only truly ambulatory system and it does not restrict the patients' activities. However, 48hours may not be long enough to capture reflux episodes that can provide important data to guide diagnosis. Furthermore, pH studies generally test only for acid reflux and therefore miss non-acid and/or bile reflux conditions.

Although the aforementioned techniques can guide management, these methods are known in the literature to have less than optimal sensitivities, and are not predictive for all reflux conditions. Detection is missed in a significant number of patients. Many patients do not have the classic presentation of heartburn, regurgitation, but rather will have vague symptoms of cough, hoarseness, postnasal drip, asthma, and chest pain.

Many scoring systems have been developed to correlate symptoms with acid reflux episodes to address these issues and establish a probability score for reflux episodes. Unfortunately, although this is highly predictive for classical symptoms such as heartburn, it is not as accurate or predictive for non-classical symptoms such as chest pain, hoarseness, cough, asthma, and postnasal drip among others. Given the uncertainty in diagnosis, when patients with non-classical symptoms undergo medical management, the success rate is only 80%. Currently, the only definitive method to determine correlation in non-classically presenting patients is surgery, because gastric contents are physically blocked from retrograde flow into the esophagus. Surgery, however, is difficult to reverse and has an associated risk of side effects and/or complications.

Acid reflux is caused when the lower esophageal sphincter malfunctions either by not closing fully, or being weak in its closure. If a functioning replacement valve could be installed, retrograde flow of acid from the stomach into the esophagus would be prevented, thus alleviating symptoms associated with the condition.

Document US2002032487 disclose an esophageal stent comprising an anchoring structure with prongs oriented both distally and proximally towards the ends of the stent.

### Summary

The present invention describes an esophageal stent including an anchoring structure and a valve.

According to a first aspect of the invention, there is provided an esophageal stent including a stent body having a proximal portion and a distal portion. The proximal portion includes an anchoring structure, the anchoring structure including a plurality of prongs each oriented towards the distal portion. The stent also includes a valve attached to the distal portion of the esophageal stent, wherein the esophageal stent (10) does not include proximally-oriented or horizontal spikes.

The anchoring structure and the stent body may separable.

The anchoring structure and the stent body may be unitary.

The anchoring structure of the proximal portion may have a larger diameter than the distal portion.

The anchoring structure of the proximal portion may comprise a T-shaped cross-section having a larger diameter than the distal portion; the proximal portion folding back on itself so as to create a hook cross-section having a larger diameter than the distal portion.

The valve may be a two-way valve.

The valve may be a split dome valve.

The valve may have at least one of an antegrade break pressure of 0-15 mmHg, a retrograde belch break pressure of 2-25 mmHg, or a retrograde vomit break pressure of 15-65 mmHg.

The length of the distal portion of the stent may be approximately a same length as an esophageal sphincter of a patient into which the stent is placed. The length of the stent body is between 0.5 cm and 5.0 cm.

The length of the stent body may be between 2.0 cm and 3.0 cm.

The distal portion may include an anchoring structure.

The anchoring structure of the distal portion may have a larger diameter than a remainder of the distal portion of the stent body.

The valve and the stent body may be integrated into a single part using overmolding.

The stent may further include a pH sensor.

The stent may include a power source operably coupled to the pH sensor and at least one of a processor, a data storage unit, or a transmitter.

The pH sensor may be located within an interior volume of the esophageal stent.

The stent may include a grip attached to the proximal portion of the stent body.

A diameter of the distal portion of the stent body may be approximately equal to an inner diameter of an esophageal sphincter of a patient into which the stent is placed.

According to a second aspect of the invention, there is provided a stent delivery device, including an outer tube configured to: house the esophageal stent of the first aspect of the invention within an inner volume of the outer tube at a distal end of the outer tube; and receive an inner tube into the inner volume of the outer tube at a proximal end of the outer tube. The inner tube is slideable within the outer tube. The delivery device also includes a tip attached to the distal end of the outer tube, the tip configured to open and allow passage of the esophageal stent out of the inner volume of the outer tube.

### Brief Description of the Drawings

FIG. 1 depicts an esophageal stent including distally-oriented prongs.
FIG. 2 depicts the esophageal stent of FIG. 1 additionally including a valve having magnet/ferromagnetic material pairs and flaps.
FIG. 3A shows a bottom view of the esophageal stent of FIG. 2 in which the valve structure includes magnets and flaps.
FIG. 3B shows a bottom view of the esophageal stent of FIG. 2 in which the valve structure includes magnets and strings.
FIG. 4 depicts an esophageal stent including a grip.
FIGS. 5A-5F depicts different embodiments of the esophageal stent which include different anchoring structures for maintaining the position of the esophageal stent.
FIG. 6 depicts an esophageal stent including a hook and valve.
FIG. 7A is a side view of a collapsed anchoring structure.
FIG. 7B is a side view of a partially collapsed anchoring structure.
FIG. 7C is a side view of an expanded anchoring structure.
FIG. 7D is a top view of the expanded anchoring structure of FIG. 7C.
FIG. 8A depicts an esophageal stent placed in the esophagus of a patient.
FIG. 8B depicts an esophageal stent including an internal valve.
FIG. 8C depicts an esophageal stent in which the valve and a coating of the stent are molded as a single process.
FIG. 8D depicts the hinged valve of the esophageal stent of FIG. 8C.
FIGS. 89-9C depict esophageal stents including different anchoring structures and valves.

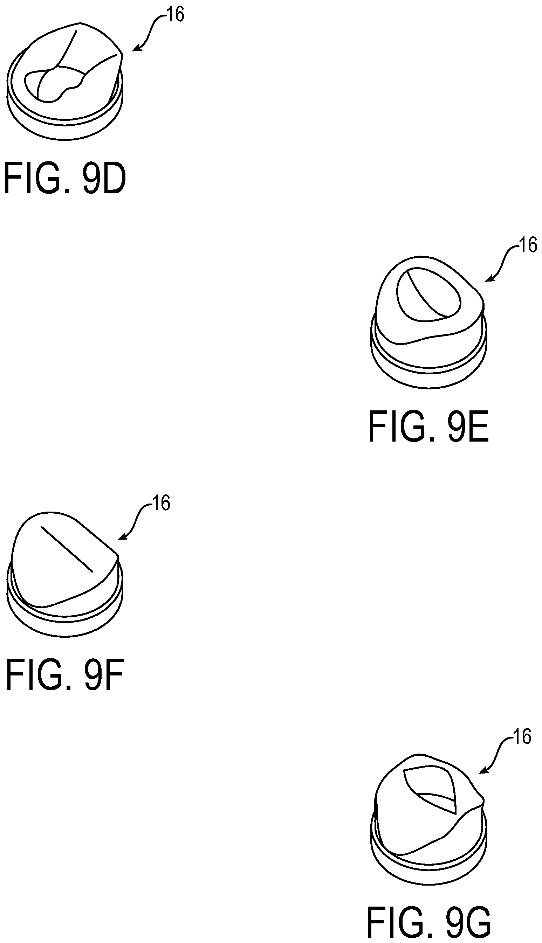

FIGS. 9D-9G depict a domed valve in different configurations.

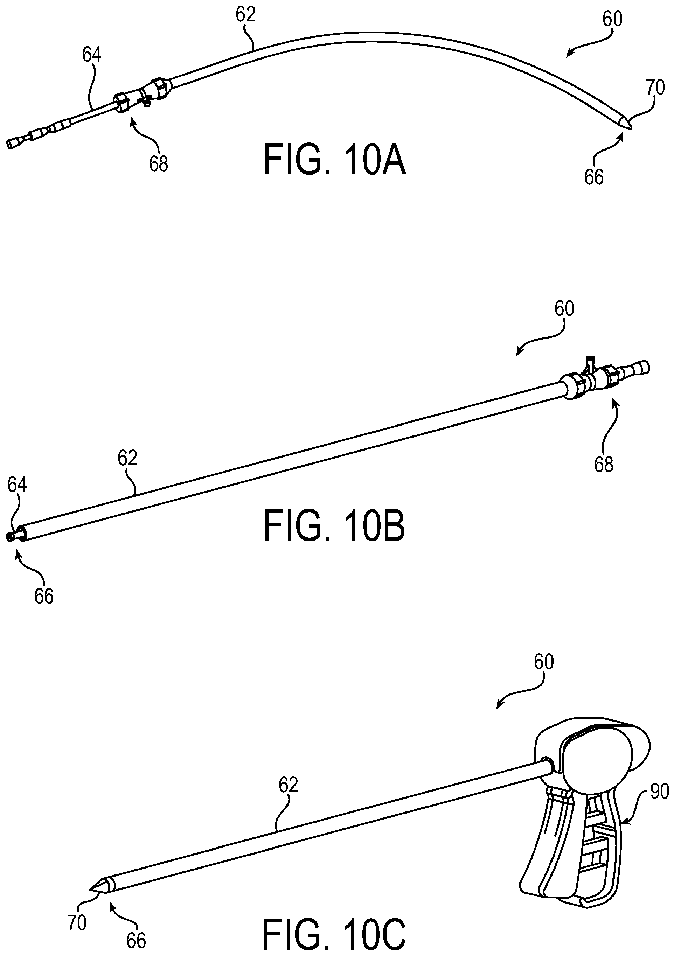

FIGS. 10A-10C depict a stent and valve system provided in a kit.

### Detailed Description

In the following description, reference is made to the accompanying drawings that form a part hereof, and in which is shown by way of illustration specific embodiments which may be practiced. These embodiments are described in detail to enable those skilled in the art to practice the invention, and it is to be understood that other embodiments may be utilized and that logical changes may be made without departing from the scope of the present invention. The following description of example embodiments is, therefore, not to be taken in a limited sense, and the scope of the present invention is defined by the appended claims.

The Abstract is provided to comply with 37 C.F.R. §1.72(b) to allow the reader to quickly ascertain the nature and gist of the technical disclosure. The Abstract is submitted with the understanding that it will not be used to interpret or limit the scope or meaning of the claims.

We disclose herein an antireflux device that is placed temporarily at the gastroesophageal junction, for example, and without limitation, a period up to, e.g., 29 days. This device enables diagnostic evaluation of patients with suspected gastric reflux and may establish a more complete understanding of the severity of reflux and the need for further management. After placement of the device, the patient can resume regular activities while the stent is in place. The device includes a one-way or a two-way valve that is designed to allow for ease of swallowing with minimal restriction while also preventing or restricting retrograde flow of gastric contents into the esophagus. If configured with a two-way vale, the valve is designed such that gastric acid is prevented from entering the esophagus, but when gastric pressure is sufficiently high from the need to belch or vomit, the valve opens to allow such activity. By restricting gastric acid from entering the esophagus, the valve eliminates or substantially reduces gastroesophageal reflux while the device is in place. As such, a patient's symptoms should improve during this time if they are due to gastroesophageal reflux. If the symptoms do not improve, clinicians are informed of the possibility of alternate causes for the symptoms.

While the device is in place, data is gathered relating to the patient's condition. Patients may, e.g., self-report the data in the form of symptoms that were experienced or alleviated during placement. Alternatively, the device may incorporate sensors, such as pH or pressure sensors, which may be reported in real time, queried, or stored in memory. The device allows the clinician or team of clinicians treating the patient to assess the patient's symptoms, or alleviation thereof, and any corresponding data gathered from sensors. With this data, the clinician or team of clinicians can more effectively determine if the patient's symptoms are due to gastroesophageal reflux and, based on this information, determine the appropriate treatment. In severe cases, the data will inform the clinician as to whether a definitive, irreversible surgical therapy that will also block flow of gastric contents into the esophagus will be effective and appropriate. After the temporary period, the device is removed and care is administered appropriately as informed, at least in part, by the data gathered.

Stents appropriate for use in the esophagus and methods to deploy them are known in the art, and are described in, for example, published PCT application WO 2013066617 titled, "Esophageal stent with valve"; US Patent 7993410 titled "Esophageal stent"; US Patent 6302917 titled "Anti-reflux esophageal prosthesis"; and US Patent 6,790,237 titled "Medical stent with a valve and related methods of manufacturing".

The design of the stent is sufficient to withstand and appropriately distribute forces experienced in the esophagus.

The stent may comprise a metal, such as stainless steel, or a shape-memory material such as nitinol, as is known in the art. The stent may alternatively comprise a polymer and/or a dissolving or degrading material, such as is known in the art, and is described, for purposes of providing an example only, in US Patent 6,537,312 entitled "Biodegradable : stent". Dissolvable and/or degradable materials eliminate the need for a second endoscopic procedure to remove the stent. As the reflux device breaks down, it is excreted from the patient safely and prevents obstruction, which is sometimes encountered with migrated metal stents. The system may be 100% dissolvable/degradable or may comprise dissolvable/degradable linkers to components that will not significantly break down, which allow the stent to break apart for and be passed by normal excretion. The stent may also contain elements, comprised of, or coated with barium or other radiopaque material to facilitate medical imaging procedures to locate components in the patient.

The length of the stent may be chosen to be larger than, but approximately equal to the length of the esophageal sphincter of the patient in which the stent will be placed. In this way, the stent will not interfere with the normal operation of (e.g., esophageal motility) of the esophagus.

The stent may be coated to facilitate deployment, as is known in the art. For example, the stent may be coated with an anti-friction coating or microtexturing.

Several features distinguish the stents of the invention. All, several, or one of these features may be used on the same stent.

The stent may also comprise sensors for fluid flow, pH, or other clinically relevant parameters. The stent may also include a power source, a data storage unit, a transmitter, and/or a processor, each of which is operably linked as is known in the art.

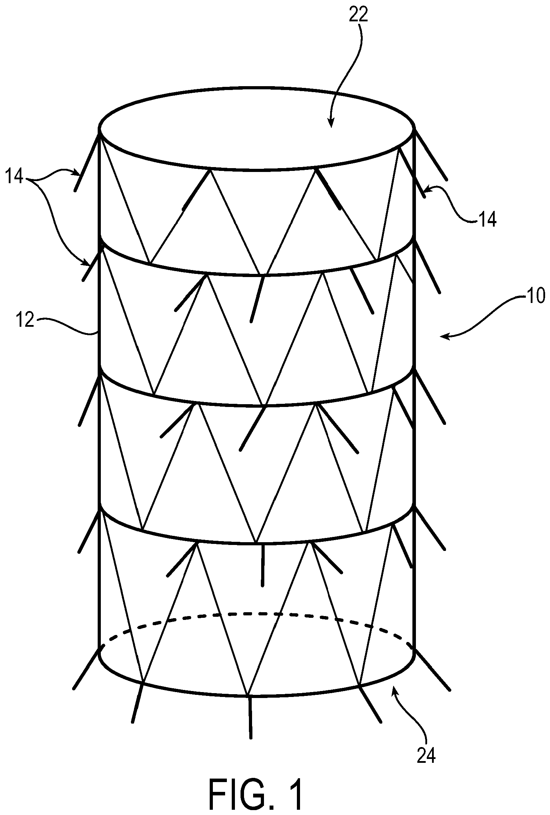

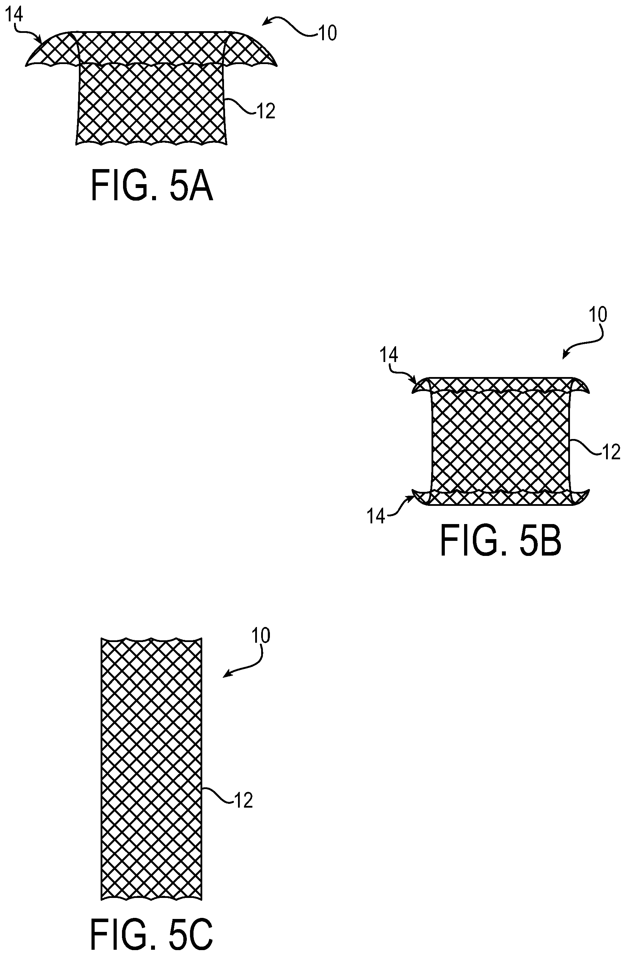

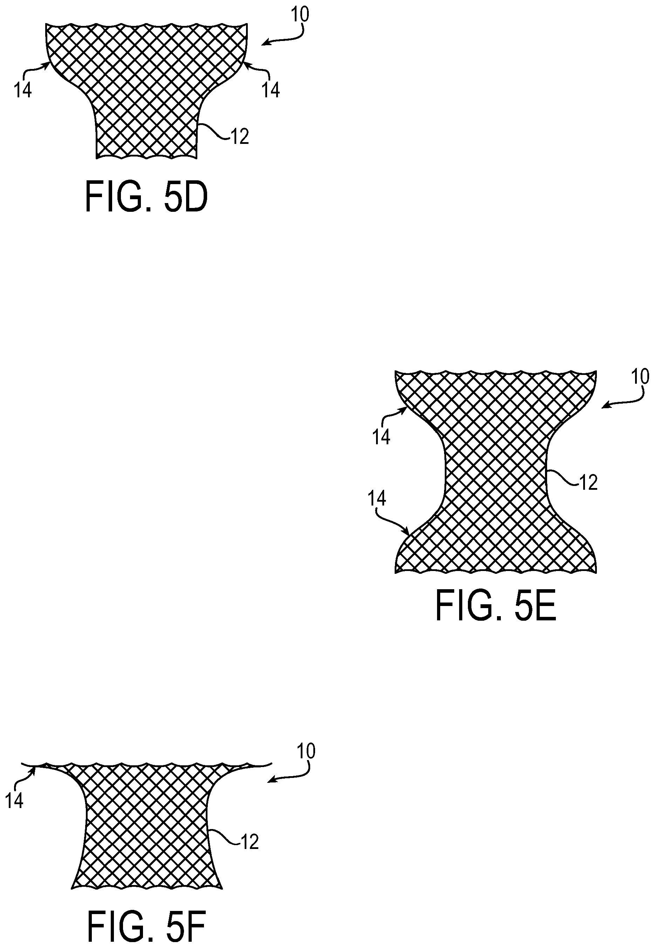

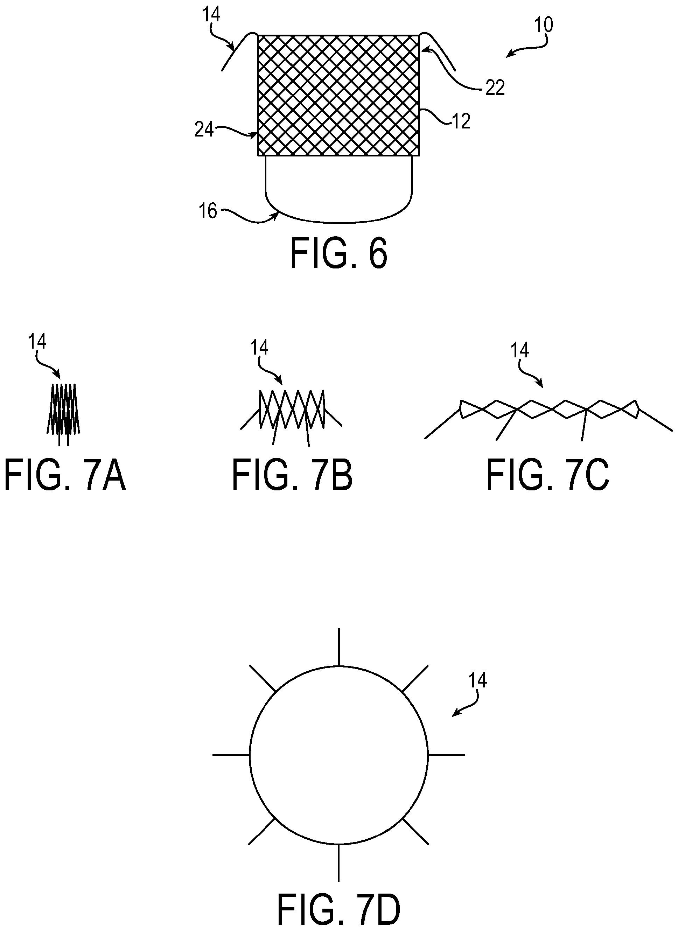

Turning to FIGS. 1 and 5, the stent 10 according to the invention includes a stent body 12, anchoring structures 14, and a valve 16. The anchoring structures 14 comprises distally-oriented prongs. An example, as shown in FIGS. 5A, 5B, 5D-F, the stent 10 may be configured in a preformed shape in which one or both ends are folded back onto itself or bulge outwards to form the anchoring structure 14. For example, as shown in FIG. 5F, the anchoring structure 24 may comprise a T-shaped cross-section having a larger diameter than the distal portion 24. Alternatively or additionally, as shown in FIG. 6, the anchoring structure 24 may comprise the proximal portion 22 folding back on itself so as to create a hook cross-section having a larger diameter than the distal portion 24.

The distally-oriented prongs and/or preformed shape of the stent 10 may serve to anchor the stent 10 in the esophagus 20. The prongs are, e.g., configured to embed into the esophagus 20 (e.g., into the submucosa of the esophagus) in order to prevent distal stent migration. The stent 10 of the invention does not include upward or horizontal spikes as to allow ease and safety of retrieval of the device when the diagnostic procedure is completed.

The stent body 12 includes a proximal portion 22 and a distal portion 24. The length of the stent body 12 may be between 0.5 cm and 5.0 cm or between 2.0 cm and 3.0 cm. The length of the distal portion 24 of the stent 10 may be approximately a same length as an esophageal sphincter of a patient into which the stent 10 is placed. For example, the length of the distal portion 24 may be 5%, 10%, or 20% of the length of the esophageal sphincter of the patient. A diameter of the distal portion 24 of the stent body 12 may be approximately equal to an inner diameter of an esophageal sphincter of a patient into which the stent 10 is placed. For example, the diameter of the distal portion 24 may be within 1%, 2.5%, 5%, or 10% of the diameter of the esophageal sphincter of the patient.

The proximal portion 22 of the stent 10 according to the invention includes include the anchoring structure 14. When the proximal portion 22 includes an anchoring structure 14, the anchoring structure 14 of the proximal portion 22 may have a larger diameter than the distal portion 24. In an example, when the distal portion 24 includes an anchoring structure 14, the anchoring structure 14 of the distal portion 24 may have a larger diameter than a remainder of the distal portion 24 of the stent body 12.

The distal portion 24 of the stent according to the present invention includes a valve 16. The valve 16 and the stent body 12 may be integrated into a single part. For example, the valve 16 and the stent body 12 may be integrated into a single part using overmolding or any other suitable technique.

The stent 10 and stent body 12 may be made of a shape memory material, configured with various preformed shapes. The stent 10 may have the ability to collapse to a substantially smaller diameter under a radially applied force, returning to the preformed shape upon removal of said force. The stent 10 shape may be a straight cylinder (FIG. 5C) that relies on the traditional radial forces typically generated from an expanding esophageal stent 10 in order to maintain the position of the stent. The stent 10 may also be a dumbbell shape with larger diameters on each end relative to the center section (FIG. 5D and 5E), T-shaped in cross-section with a larger diameter on one end relative to the center section (FIG. 5F), conical, or may be folded back on itself on one end as to create a hook cross-section on one or both ends (FIG. 5A, 5B). The hook cross-section is configured to reduce propagation of the stent 10 once placed. As described above, the stent 10 may also include one or more features, such as prongs attached to the stent 10 body to reduce propagation.

Figure 1 shows an embodiment of the location and number of distally-oriented prongs acting as anchoring structures 14. The number and location of said prongs may be varied; the exact number, and the lateral and vertical spacing of said prongs are also variable. For example, there may be anywhere from 1 to 100 prongs on any lateral ring, and as many 1 to 100 prongs in any vertical along the stent 10 face.

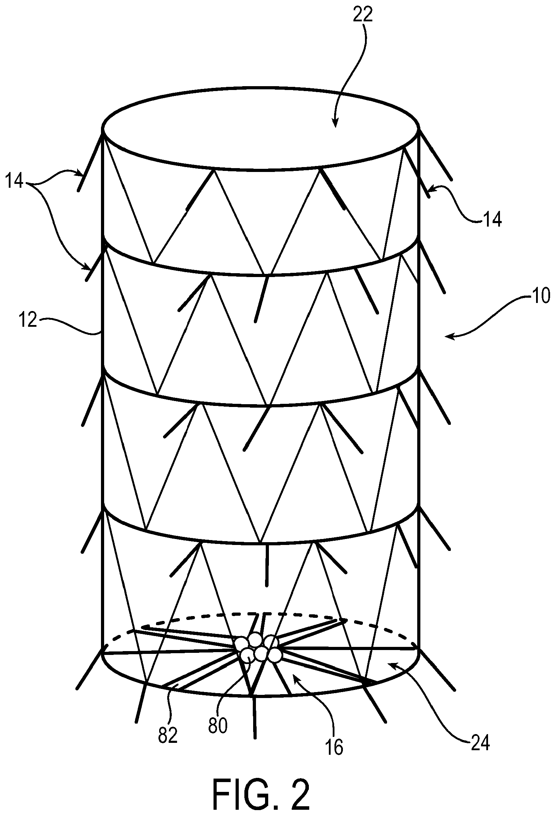

In FIGS. 1 and 2 the anchoring structure 14 and the stent 10 body are unitary in construction. That is, the anchoring structure 14 and the stent 10 body are integral with one another. As shown in FIGS. 7A-7D, the anchoring structure 14 may instead be separable/separated from the stent body 12. In this embodiment, the stent 10 body is separable from the anchoring structure 14 as a physically distinct object. For example, in this embodiment, the stent 10 may be first placed in a patient. Once the stent 10 is in place, the anchoring structure 14 may be placed in the patient in order to maintain the position of the stent 10. The stent 10 may or may not include a valve 24. For example, the stent 10 may include a valve 24 without the anchoring structure 14.

Turning to FIGS. 7A-7D, the anchoring structure 14 may be formed as a collapsible ring including prongs for maintaining the position of the anchoring structure 14. The anchoring structure 14 may be collapsed as shown in FIG. 7A when the anchoring structure 14 is being placed within a patient. Once in position, the anchoring structure 14 may be expanded or allowed to expand from the collapsed state shown in FIG. 7A, to a partially collapsed state shown in FIG. 7B, and into an open (i.e., expanded) state shown in FIG. 7C where the prongs of the anchoring structure 14 maintain the position of the anchoring structure 14. As shown in FIG. 7D, when expanded, the anchoring structure 14 may be circular in shape with prongs extending outwards from the circular shape. The anchoring structure 14 may be positioned within the interior volume of the esophageal stent 10, such that when the anchoring structure 14 expands, the prongs of the anchoring structure 14 pass through the wall of the stent 10 body and anchor in the surrounding tissue. For example, the stent 10 body may be a mesh and the prongs of the anchoring structure 14 may pass through the gaps in the mesh in order to anchor the esophageal stent 10.

As will be understood by one of ordinary skill in the art, the anchoring structure 14 may be comprised of self-expanding nitinol, shape memory material, super elastic material, or balloon expanded stainless steel.

The anchoring structure 14 may be formed as a singular ring when expanded. The anchoring structure 14 may also be formed from multiple interconnected rings when expanded.

The stent 10 may be encompassed by a sheath, in order to facilitate the procedure, through which the stent 10 is placed. The sheath may hold the distally-oriented prongs flat, at least in line with the stent 10 surface, such that stent 10 may smoothly be moved into position. Once the stent 10 is near its final location, the sheath may be withdrawn proximally, freeing the distally-oriented prongs, which are configured to spring radially outward. Once the sheath is fully withdrawn, the sheath may be advanced distally, at a distance sufficient to embed the distally-oriented prongs into the esophageal submucosa or wall.

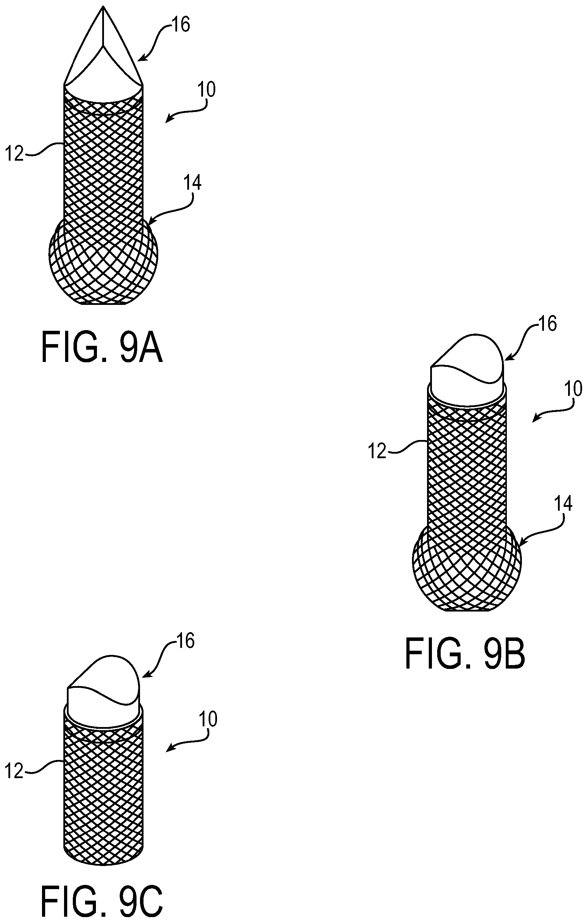

A valve 16 may be attached to the stent 10 and regulate flow into and/or out of the esophagus. The valve 16 may be a one-way or check valve (FIG. 9A) that prevents retrograde flow from the stomach into the esophagus. Said one-way valve may be an umbrella valve, duckbill valve, or it may be a cross-slit valve, or it may be a swing or tilting disc valve, or it may be a diaphragm valve. As shown in FIGS. 9B-9G, the valve may alternatively be a two-way valve that restricts retrograde flow from the stomach into the esophagus, but allows for belching and/or vomiting. For example, FIG. 9D shows the valve retrograde open, FIG. 9E shows the valve 16 retrograde closed, FIGS. 9B, 9C, and 9F show the valve 16 in nominal shape, and FIG. 9G shows the valve 16 antegrade open.

The two-way valve may be a dome valve, a split dome vale, or it may be a combination valve such as an umbrella/duckbill combination valve, or it may be two one-way valves. Said one-way or two-way valve may be formed of one or more thermoplastic or thermoset materials, or may contain magnets as a non-permanent means of closing the valve. The valve may also contain one or more additives or molded features as to prevent or slow microbial and/or biofilm growth or accumulation. Friction reducing surface coatings (e.g., parylene) or microtexturing may also be added to the valve.

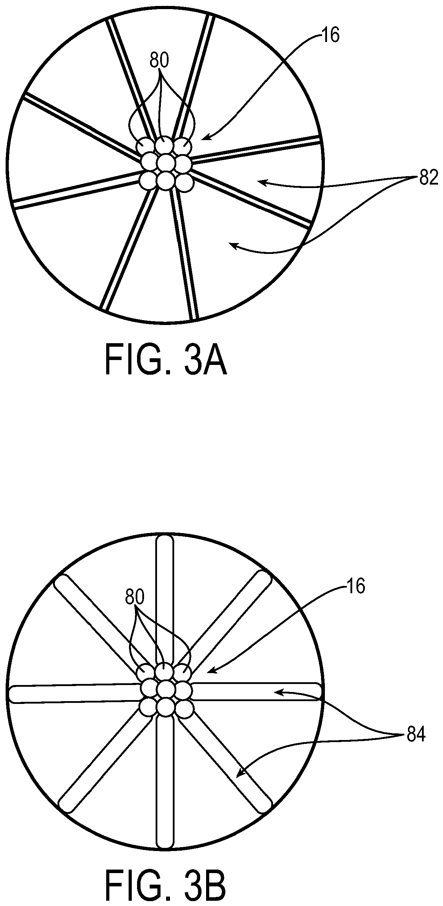

In another embodiment, the use of a reflux barrier valve may incorporate magnets 80 to secure the valve closing as shown in FIGS. 2, 3A, and 3B. The valve 16 may be secured to the distal end 24 of the stent 10, within the stent 10, or at the proximal end 22 of the stent 10. The valve 16 may comprise permanent magnet and/or ferromagnetic material pairs to non-permanently close the stent 10 to prevent reflux after swallowing. As shown in FIGS. 2 and 3A, the magnet/ferromagnetic material pairs 80 may be attached to the end of flaps 82. As shown in Figure 3B, the magnet/ferromagnetic material pairs 80 may be attached to the end of strips or strings 84.

The valve 16 may be bicuspid, tricuspid, or have more than three components. The valve 16 may also be a one-way or two-way valve or a combination of one-way valves. If configured with a one-way valve, the valve 16 may be selected from any of the following types: umbrella valve, duckbill valve, cross-slit valve, swing or tilting disc valve, or diaphragm valve. If configured with a two-way valve, the valve 16 may be selected from any of the following types: dome valve, combination valve such as an umbrella/duckbill combination valve. The valve 16 may also be two or more one-way valves. The valve 16 may be designed so as to allow relatively unrestricted antegrade flow, while either preventing (one-way) or restricting (two-way) retrograde flow. If the valve 16 is configured to restrict flow, the valve 16 may be designed such as to allow retrograde flow once gastric pressure reaches between 10mm to 40mm Hg similar for vomiting and/or belching. For example, the valve 16 may have at least one of an antegrade break pressure of 0-15 mmHg, a retrograde belch break pressure of 2-25 mmHg, or a retrograde vomit break pressure of 15-65 mmHg.

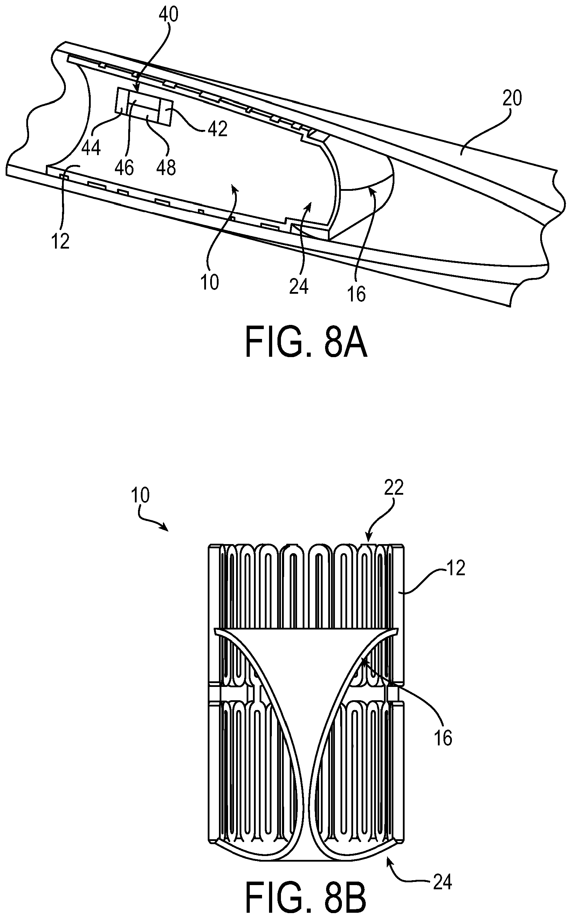

Turning to FIG. 8A, the stent 10 is shown inside the esophagus 20 of a patient. As shown in the figure, the stent 10 may additionally include a pH sensor 40. A power source 42 may be operably coupled to the pH sensor 40 and at least one of a processor 44, a data storage unit 46, or a transmitter 48. The pH sensor may be located within an interior volume of the esophageal stent 10.

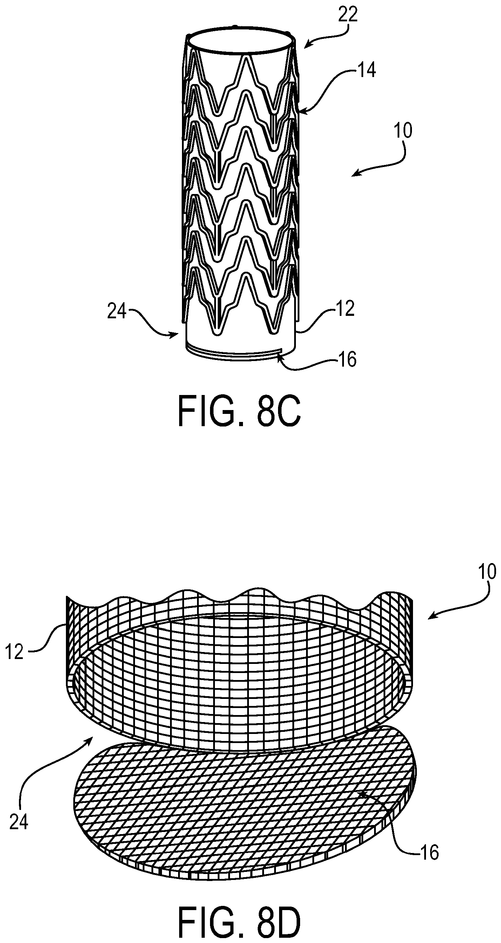

Turning to FIG. 8B, the valve 16 may be internal to the stent 10. FIG. 8B shows a cross sectional view of an exemplary internal valve 16. The valve 16 may be shaped such that it requires less force to go through the valve in a downward direction (relative to the placement of the valve as shown in the figure) as compared to the force required to pass through the valve in an upwards direction. The valve 16 may also be a hinged one way valve as shown in FIGS. 8C and 8D.

The valve material is selected to withstand degradation from the high acid content of the stomach; such materials are known in the art. Suitable materials, which may be unfilled, include natural rubbers such as Hevea and thermoplastic, i.e., melt-processible, or thermosetting, i.e., vulcanizable, synthetic rubbers such as: fluoro- or perfluoroelastomers, chlorosulfonate, polybutadiene, butyl, neoprene, nitrite, polyisoprene, buna-N, copolymer rubbers such as ethylenepropylene (EPR), ethyl ene-propylene-diene monomer (EPDM), acrylonitrile-butadiene (NBR or FTNBR) and styrene-butadiene (SBR), and blends such as ethylene or propylene-EPDM, EPR, or NBR. The term "synthetic rubbers" also should be understood to encompass materials which alternatively may be classified broadly as thermoplastic or thermosetting elastomers such as polyurethanes, silicones, fluorosilicones, styrene-isoprene-styrene (SIS), and styrene-butadiene-styrene (SBS), as well as other polymers which exhibit rubberlike properties such as plasticized nylons, polyolefms, polyesters, ethylene vinyl acetates, fluoropolymers, and polyvinyl chloride. The material may be formed of one or more thermoplastic or thermoset materials, or may contain magnets as a non-permanent means of closing the valve. The valve 16 may also contain one or more additives or molded features as to prevent or slow microbial or biofilm growth or other accumulation. The valve 16 may also include friction reducing surfaces on the valve to aid the deployment of the stent 10.

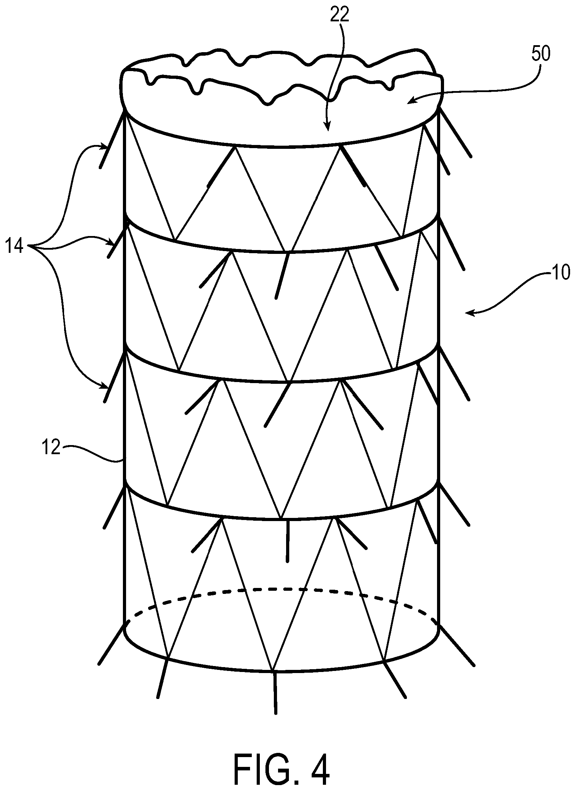

As is shown in Figure 4, the stent 10 may comprise a grip 50 attached to the proximal portion 22 of the stent body 12. The grip 50 may function to provide a convenient surface to grip, in order to withdraw the device from the patient. The grip 50 may be a continuous surface at the proximal end 22 of the device as shown Figure 4, and may be affixed to the stent 10 or integral to it, or may comprise one or more discontinuous proximal extensions. The grip 50 may extend proximally, as shown in Figure 4, or may extend inward, or distally, or any variation thereof. The grip 50 may also be affixed or integral to the stent 10 at a location distal to the proximal end 22. The grip 50 may comprise a string or any suitable structure by which the stent 10 may be pulled through the esophagus 20 of the patient during removal of the stent 10. When pulled, the grip 50 may act to reduce the diameter of the stent 10, making it easier to remove the stent 10.

The stent 10 may be provided with a sheath to be used during a procedure to extract the stent 10. The sheath is advanced into the esophagus until it is just proximal to the stent 10, or until it reaches the most proximal prongs. As the stent 10 is withdrawn, the stent 10 slides inside the sheath, forcing the distally-oriented prongs to bend inward, in line with the stent 10 face. The stent 10 may be further withdrawn, or the sheath further advanced distally until the next set of prongs is reached, and the stent 10 is withdrawn until the stent 10 is encompassed by the sheath, which is then withdrawn from the esophagus together with the stent 10, thus protecting the esophagus from the prongs as the stent 10 is withdrawn.

The stent 10 and valve system may be provided in a kit. As shown in FIGS. 10A-10C, the kit may further comprise a stent delivery device 80 for placing the stent 10 and valve 16 into the lower esophagus. As shown in FIG. 10A, the device 80 may comprise an outer tube 62 configured to house the esophageal stent 10 within an inner volume of the outer tube 62 at a distal end 66 of the outer tube 62. The outer tube 62 is configured to receive an inner tube 64 (e.g., a plunger) within the inner volume of the outer tube 62 at a proximal end 68 of the outer tube 64. The inner tube 64 is slideable within the outer tube 64, such that the inner tube 64 may be activated by a clinician to deploy the stent 10 into place in the esophagus. The inner tube 64 and/or an inner diameter of the outer tube 62 may include friction reducing surface structures. The device 80 may be equipped with a means of retracting the flexible tube while keeping the stent 10 in place, exposing the device for deployment upon retraction of the outer tube 62 as shown in FIG. 10B.

Turning to FIG. 10C, the device 80 may also include a tip 70 attached to the distal end 66 of the outer tube 62. The tip 70 is configured to open and allow passage of the esophageal stent 10 out of the inner volume of the outer tube 74. The tip may be a flexible polymeric tip configured to have a relative point at the proximal end to guide the device through the esophagus and through the lower esophageal sphincter as desired. Said polymeric tip 70 may be affixed to the outer diameter of the flexible tube, in which case, the polymeric tip is split, allowing the stent 10 to exit through the tip 70 upon deployment in the esophagus, or it may be affixed to an inner, more rigid component configured within the flexible tube, in which case, the polymeric tip 70 is solid, and stays in place when the flexible tube is retracted, exposing the stent 10 and valve system for deployment.

With further reference to FIG. 10C, the device for placing the stent 10 may additionally include a handle 90. When squeezed, the handle 90 advances the inner tube 64 such that the esophageal stent 10 is pushed through the tip 70. The handle 90 may allow the user to push the inner tube 64 forward in discreet increments, e.g., using a ratcheting action.

The stent 10 may be deployed (e.g., with a sheath) down the esophagus endoscopically, as is known in the art. If a sheath is used, it is removed, and the stent 10 is advanced distally sufficiently to anchor it. The stent '0 is left in place temporarily, for example, between 7 and 90 days, or between 14 and 30 days, or 29 days. If one or more sensors are incorporated into the device, data may be collected, and if one or more transmitters are incorporated into the device, data may be collected from the stent 10 during the diagnostic period, for example, by transmission to a receiver, which may be a smart phone, and said receiver may in turn relay the information to a third party, such as a service provider or physician. At the end of the diagnostic period, the stent 10 may be withdrawn endoscopically or via other procedure. A sheath may be used during said procedure to protect the esophagus from the prongs. The device may also include one or more dissolvable and/or degradable components such that it falls apart and is excreted by the patient after the diagnostic period has ended.

The stent 10 and or the device for placing the stent 10 may include radio frequency identification (RFID) technology. For example, the properties of the stent 10 and or the device for placing the stent 10 may be determined using an RFID reader. As an example, the length of a stent 10 and/or a diameter of the stent 10 may be determined using an RFID reader.

A method for diagnosing gastric reflux in a patient using an esophageal stent 10 including a stent body 12, a valve 16, and an anchoring structure 14 is described. In the method, the esophageal stent 10 is placed within the inner volume of an esophageal sphincter of the patient. The stent 10 is placed such that the valve 16 of the stent 10 extends past the esophageal sphincter and into the inner volume of a stomach of the patient. The valve 16 prevents the retrograde movement of fluid from the stomach into an esophagus of the patient. The stent 10 is also placed such that the anchoring structure 14 of the stent 10 is proximal to the esophageal sphincter.

The method may also include removing the esophageal stent 10 from the esophagus of the patient a temporary period has expired. The temporary period may be between 7 and 90 days, between 14 and 30 days, or 29 days.

The method may also include gathering data for diagnosing whether the patient has acid reflux during a time over which the esophageal stent 10 is located within the esophagus of the patient. For example, the data may be gathered using the pH sensor 40.

The method may also include removing the stent 10 by retrograde withdrawal of the stent 10 through the esophagus of the patient. Alternatively or additionally, the esophageal stent 10 is made of one or more dissolvable or degradable components. In this example, the stent 10 may degrade within the patient and is excreted from the patient.

In another method for diagnosing gastric reflux in a patient using the esophageal stent 10, the esophageal stent 10 is placed such that the valve 16 of the stent prevents the retrograde movement of fluid from the stomach into an esophagus of the patient. The method also includes periodically collecting data for diagnosing whether the patient has acid reflux during a time over which the esophageal stent 10 is located within the esophagus of the patient.

In a further method for diagnosing gastric reflux in a patient in which an esophageal stent 10 has been positioned such that the valve 16 of the stent 10 prevents the retrograde movement of fluid from the stomach into an esophagus of the patient, data is periodically collected for diagnosing whether the patient has acid reflux during a time over which the esophageal stent 10 is located within the esophagus of the patient.

The stent 10 may be deployed in subjects including human and non-human animals. Non-human animals includes all vertebrates, e.g., mammals and non-mammals, such as non-human primates, sheep, dogs, cats, cows, horses, chickens, amphibians, and reptiles, although mammals are preferred, such as non-human primates, sheep, dogs, cats, cows and horses. The subject may also be livestock such as, cattle, swine, sheep, poultry, and horses, or pets, such as dogs and cats.

Preferred subjects may include human subjects having one or more symptoms of gastric reflux.

The present invention is limited by the scope of the following claims.

## Claims

1. An esophageal stent (10) comprising:
a stent body (12) having a proximal portion (22) and a distal portion (24), wherein the proximal portion (22) includes an anchoring structure (14), the anchoring structure (14) including a plurality of prongs each oriented towards the distal portion; and
a valve (16) attached to the distal portion (24) of the esophageal stent (10),
wherein the esophageal stent (10) does not include proximally-oriented or horizontal spikes.

2. The esophageal stent (10) of any of the preceding claims, wherein the anchoring structure (14) and the stent body (12) are separable.

3. The esophageal stent (10) of any of the preceding claims, wherein the anchoring structure (14) and the stent body (12) are unitary.

4. The esophageal stent (10) of any of the preceding claims, wherein the anchoring structure (14) of the proximal portion (22) has a larger diameter than the distal portion (24).

5. The esophageal stent (10) of any of the preceding claims, wherein the valve (16) is at least one of a two-way valve or a split dome valve.

6. The esophageal stent (10) of any of the preceding claims, wherein the valve (16) has at least one of an antegrade break pressure of 0-15 mmHg, a retrograde belch break pressure of 2-25 mmHg, or a retrograde vomit break pressure of 15-65 mmHg.

7. The esophageal stent (10) of any of the preceding claims, wherein the length of the distal portion (24) of the stent (10) is approximately a same length as an esophageal sphincter of a patient into which the stent (10) is placed.

8. The esophageal stent (10) of any of the preceding claims, wherein the length of the stent body (12) is between 0.5 cm and 5.0 cm.

9. The esophageal stent (10) of claim 6, wherein the anchoring structure (14) of the distal portion (24) has a larger diameter than a remainder of the distal portion (24) of the stent body (12).

10. The esophageal stent (10) of any of the preceding claims, wherein the valve (16) and the stent body (12) are integrated into a single part using overmolding.

11. The esophageal stent (10) of any of the preceding claims, further comprising a pH sensor.

12. The esophageal stent (10) of claim 11, further comprising a power source operably coupled to the pH sensor and at least one of a processor, a data storage unit, or a transmitter.

13. The esophageal stent (10) of any of the preceding claims, further comprising a grip (50) attached to the proximal portion (22) of the stent body (12).

14. The esophageal stent (10) of any of the preceding claims, wherein a diameter of the distal portion (24) of the stent body (12) is approximately equal to an inner diameter of an esophageal sphincter of a patient into which the stent (10) is placed.

15. A stent delivery device (80), comprising:
an outer tube (62) configured to:
house the esophageal stent (10) of any of the preceding claims within an inner volume of the outer tube (62) at a distal end (66) of the outer tube (62); and
receive an inner tube (64) into the inner volume of the outer tube (64) at a proximal end (68) of the outer tube (64);
the inner tube (64) slideable within the outer tube (64);
a tip (70) attached to the distal end (66) of the outer tube (62), the tip (70) configured to open and allow passage of the esophageal stent (10) out of the inner volume of the outer tube (74).

## Patentansprüche

1. Ösophagusstent (10), umfassend:
einen Stentkörper (12) mit einem proximalen Abschnitt (22) und einem distalen Abschnitt (24), wobei der proximale Abschnitt (22) eine Verankerungsstruktur (14) beinhaltet, wobei die Verankerungsstruktur (14) mehrere Ausläufer beinhaltet, die jeweils zu dem distalen Abschnitt hin ausgerichtet sind; und
ein Ventil (16), das an dem distalen Abschnitt (24) des Ösophagusstents (10) angebracht ist,
wobei der Ösophagusstent (10) keine proximal ausgerichteten oder horizontalen Zacken beinhaltet.

2. Ösophagusstent (10) nach einem der vorhergehenden Ansprüche, wobei die Verankerungsstruktur (14) und der Stentkörper (12) trennbar sind.

3. Ösophagusstent (10) nach einem der vorhergehenden Ansprüche, wobei die Verankerungsstruktur (14) und der Stentkörper (12) unitär sind.

4. Ösophagusstent (10) nach einem der vorhergehenden Ansprüche, wobei die Verankerungsstruktur (14) des proximalen Abschnitts (22) einen größeren Durchmesser als der distale Abschnitt (24) aufweist.

5. Ösophagusstent (10) nach einem der vorhergehenden Ansprüche, wobei das Ventil (16) mindestens eines von einem Zweiwegeventil oder einem geteilten Domventil ist.

6. Ösophagusstent (10) nach einem der vorhergehenden Ansprüche, wobei das Ventil (16) mindestens einen von einem antegraden Öffnungsdruck von 0-15 mmHg, einem retrograden Aufstoßen-Öffnungsdruck von 2-25 mmHg oder einem retrograden Erbrechen-Öffnungsdruck von 15-65 mmHg aufweist.

7. Ösophagusstent (10) nach einem der vorhergehenden Ansprüche, wobei die Länge des distalen Abschnitts (24) des Stents (10) ungefähr dieselbe Länge wie ein Ösophagussphinkter eines Patienten, in dem der Stent (10) platziert wird, aufweist.

8. Ösophagusstent (10) nach einem der vorhergehenden Ansprüche, wobei die Länge des Stentkörpers (12) zwischen 0,5 cm und 5,0 cm liegt.

9. Ösophagusstent (10) nach Anspruch 6, wobei die Verankerungsstruktur (14) des distalen Abschnitts (24) einen größeren Durchmesser als ein Rest des distalen Abschnitts (24) des Stentkörpers (12) aufweist.

10. Ösophagusstent (10) nach einem der vorhergehenden Ansprüche, wobei das Ventil (16) und der Stentkörper (12) unter Verwendung von Überspritzung in ein einziges Teil integriert werden.

11. Ösophagusstent (10) nach einem der vorhergehenden Ansprüche, weiterhin umfassend einen pH-Sensor.

12. Ösophagusstent (10) nach Anspruch 11, weiterhin umfassend eine Energiequelle, die funktionsfähig an den pH-Sensor gekoppelt ist, und mindestens einen von einem Prozessor, einer Datenspeichereinheit oder einem Transmitter.

13. Ösophagusstent (10) nach einem der vorhergehenden Ansprüche, weiterhin umfassend einen Griff (50), der an den proximalen Abschnitt (22) des Stentkörpers (12) angebracht ist.

14. Ösophagusstent (10) nach einem der vorhergehenden Ansprüche, wobei ein Durchmesser des distalen Abschnitts (24) des Stentkörpers (12) ungefähr gleich einem Innendurchmesser eines Ösophagussphinkters eines Patienten, in dem der Stent (10) platziert wird, ist.

15. Stentabgabevorrichtung (80), umfassend:
einen äußeren Schlauch (62), der konfiguriert ist zum: Unterbringen des Ösophagusstents (10) nach einem der vorhergehenden Ansprüche innerhalb eines Innenvolumens des äußeren Schlauchs (62) an einem distalen Ende (66) des äußeren Schlauchs (62); und
Aufnehmen eines inneren Schlauchs (64) in dem Innenvolumen des äußeren Schlauchs (64) an einem proximalen Ende (68) des äußeren Schlauchs (64);
den inneren Schlauch (64), der innerhalb des äußeren Schlauchs (64) verschiebbar ist;
eine Spitze (70), die an dem distalen Ende (66) des äußeren Schlauchs (62) angebracht ist, wobei die Spitze (70) dazu konfiguriert ist, den Ösophagusstent (10) zu öffnen und einen Durchtritt dieses aus dem Innenvolumen des äußeren Schlauchs (74) zuzulassen.

## Revendications

1. Endoprothèse œsophagienne (10) comprenant :
un corps d'endoprothèse (12) ayant une partie proximale (22) et une partie distale (24), dans lequel la partie proximale (22) comporte une structure d'ancrage (14), la structure d'ancrage (14) comportant une pluralité de pointes orientées chacune en direction de la partie distale ; et
une valve (16) fixée à la partie distale (24) de l'endoprothèse œsophagienne (10),
dans laquelle l'endoprothèse œsophagienne (10) ne comporte pas de crampons orientés en direction proximale ou horizontaux.

2. Endoprothèse œsophagienne (10) selon l'une quelconque des revendications précédentes, dans laquelle la structure d'ancrage (14) et le corps d'endoprothèse (12) sont séparables.

3. Endoprothèse œsophagienne (10) selon l'une quelconque des revendications précédentes, dans laquelle la structure d'ancrage (14) et le corps d'endoprothèse (12) sont d'une seule pièce.

4. Endoprothèse œsophagienne (10) selon l'une quelconque des revendications précédentes, dans laquelle la structure d'ancrage (14) de la partie proximale (22) a un plus grand diamètre que la partie distale (24).

5. Endoprothèse œsophagienne (10) selon l'une quelconque des revendications précédentes, dans laquelle la valve (16) est l'une au moins parmi une valve bidirectionnelle ou une valve en forme de dôme fendu.

6. Endoprothèse œsophagienne (10) selon l'une quelconque des revendications précédentes, dans laquelle la valve (16) a l'une au moins parmi une pression de rupture antérograde de 0 à 15 mmHg, une pression de rupture rétrograde en cas de remontée gastrique de 2 à 25 mmHg, ou une pression de rupture rétrograde en cas de vomissement de 15 à 65 mmHg.

7. Endoprothèse œsophagienne (10) selon l'une quelconque des revendications précédentes, dans laquelle la longueur de la partie distale (24) de l'endoprothèse (10) est approximativement une longueur identique à celle d'un sphincter œsophagien d'un patient chez lequel l'endoprothèse (10) est placée.

8. Endoprothèse œsophagienne (10) selon l'une quelconque des revendications précédentes, dans laquelle la longueur du corps d'endoprothèse (12) est entre 0,5 cm et 5,0 cm.

9. Endoprothèse œsophagienne (10) selon la revendication 6, dans laquelle la structure d'ancrage (14) de la partie distale (24) a un plus grand diamètre qu'un restant de la partie distale (24) du corps d'endoprothèse (12).

10. Endoprothèse œsophagienne (10) selon l'une quelconque des revendications précédentes, dans laquelle la valve (16) et le corps d'endoprothèse (12) sont intégrés en une pièce unique en utilisant le surmoulage.

11. Endoprothèse œsophagienne (10) selon l'une quelconque des revendications précédentes, comprenant en outre un capteur de pH.

12. Endoprothèse œsophagienne (10) selon la revendication 11, comprenant en outre une source d'énergie couplée fonctionnellement au capteur de pH et à l'un au moins parmi un processeur, une unité de stockage de données ou un transmetteur.

13. Endoprothèse œsophagienne (10) selon l'une quelconque des revendications précédentes, comprenant en outre un élément de préhension (50) fixé à la partie proximale (22) du corps d'endoprothèse (12).

14. Endoprothèse œsophagienne (10) selon l'une quelconque des revendications précédentes, dans laquelle un diamètre de la partie distale (24) du corps d'endoprothèse (12) est approximativement égal à un diamètre intérieur d'un sphincter œsophagien d'un patient chez lequel l'endoprothèse (10) est placée.

15. Dispositif de délivrance d'endoprothèse (80), comprenant :
un tube extérieur (62) configuré pour :
loger l'endoprothèse œsophagienne (10) selon l'une quelconque des revendications précédentes à l'intérieur d'un volume intérieur du tube extérieur (62) à une extrémité distale (66) du tube extérieur (62) ; et
recevoir un tube intérieur (64) dans le volume intérieur du tube extérieur (64) à une extrémité proximale (68) du tube extérieur (64) ;
le tube intérieur (64) pouvant coulisser à l'intérieur du tube extérieur (64) ;
un embout (70) fixé à l'extrémité distale (66) du tube extérieur (62), l'embout (70) étant configuré pour s'ouvrir et permettre le passage de l'endoprothèse œsophagienne (10) hors du volume intérieur du tube extérieur (74).
